(19) Europäisches Patentamt
European Patent Office
Office européen des brevets

(11) Veröffentlichungsnummer: **0 017 893**
**B1**

(12) # EUROPÄISCHE PATENTSCHRIFT

(45) Veröffentlichungstag der Patentschrift: **24.03.82**

(51) Int. Cl.³: **C 07 D 207/06,** C 07 D 211/14, C 07 D 223/04, C 07 D 241/04, C 07 D 265/30, C 07 D 295/02

(21) Anmeldenummer: **80101856.5**

(22) Anmeldetag: **08.04.80**

(54) **Verfahren zur Herstellung von heterocyclischen tertiären Aralkylaminen.**

(30) Priorität: **11.04.79 DE 2914646**
**27.09.79 DE 2939060**

(43) Veröffentlichungstag der Anmeldung: **29.10.80 Patentblatt 80/22**

(45) Bekanntmachung des Hinweises auf die Patenterteilung: **24.03.82 Patentblatt 82/12**

(84) Benannte Vertragsstaaten: **AT BE CH DE FR GB IT LI LU NL SE**

(56) Entgegenhaltungen:
**AT-B-311 969**
**DE-A1-2 656 747**
**DE-B-1 194 865**
**EP-A2-0 000 333**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Goetz, Norbert, Dr., Schoefferstrasse 25, D-6520 Worms (DE)**
Erfinder: **Hupfer, Leopold, Dr., Waltershoehe 3, D-6701 Friedelsheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Verfahren zur Herstellung von heterocyclischen tertiären Aralkylaminen.

Die vorliegende Erfindung betrifft ein Verfahren zur Herstellung von Aralkylaminen durch Umsetzung von Aralkenalen oder Aralkanalen mit sekundären Aminen.

Es ist bekannt, dass tertiäre Amine durch Umsetzung von sekundären Aminen mit Carbonylverbindungen in Gegenwart von Wasserstoff und einem Hydrierkatalysator erhalten werden können (Houben-Weyl «Methoden der organischen Chemie» 4/2, S. 328 ff, 11/1, S. 602 ff, S. 643 ff). In der deutschen Patentschrift 1 179 947 wird die Synthese N-alkylierter aromatischer Amine durch reduktive Alkylierung aromatischer Amine an einem Palladium-Silber-Katalysator unter Schonung des aromatischen Systems beschrieben. Nach DE-OS 2 118 283 kann ebenfalls mit einem Palladium-Silber-Katalysator die Herstellung von tertiären aliphatischen oder cycloaliphatischen Aminen durch Umsetzung von aliphatischen oder cycloaliphatischen Carbonylverbindungen mit sekundären Aminen in Gegenwart von Wasserstoff vorgenommen werden. Der Vorteil dieses Katalysators besteht darin, dass man mit ihm die reduktive Alkylierung von sekundären Aminen mit gesättigten Carbonylverbindungen in hoher Ausbeute und weitgehend frei von Nebenprodukten durchführen kann. Als geeignete Carbonylverbindungen werden insbesondere gesättigte Ketone, wie Aceton, Methyläthylketon, Cyclopentanon, Cyclohexanon und prinzipiell auch Aldehyde, die keine oder nur schwer Adolkondensation eingehen, wie Formaldehyd, Isobutyraldehyd, 2-Äthylhexanal aufgeführt. In der deutschen Patentanmeldung P2 830 999 wird die Herstellung von stereoisomerem N-Aralkyl-2,6-dimethylmorpholinen durch Umsetzung von stereoisomeren 2,6-di-Dimethylmorpholinen mit Carbonylverbindungen in Gegenwart von Wasserstoff und einem Palladium-Silber-Katalysator beschrieben.

Es wurde nun gefunden, dass man tertiäre Amine der Formel I

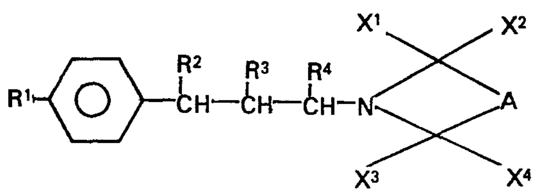

I

in der $R^1$ ein Wasserstoffatom, einen aliphatischen Rest mit 1 bis 10 Kohlenstoffatomen, einen cycloaliphatischen Rest mit 5 bis 7 Kohlenstoffatomen, eine Alkoxygruppe mit 1 bis 6 Kohlenstoffatomen, $R^2$, $R^3$ und $R^4$ ein Wasserstoffatom oder einen Alkylrest mit 1 bis 4 Kohlenstoffatomen, $X^1$, $X^2$, $X^4$ ein Wasserstoffatom oder einen Alkylrest mit 1 bis 4 Kohlenstoffatomen, A den Rest

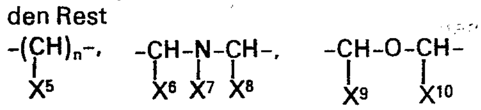

und $X^5$, $X^6$, $X^7$, $X^8$, $X^9$ und $X^{10}$ ein Wasserstoffatom oder einen Alkylrest mit 1 bis 4 Kohlenstoffatomen und n die Zahlen 1, 2, 3 oder 4 bedeutet, durch Umsetzung von sekundären Aminen der Formel II

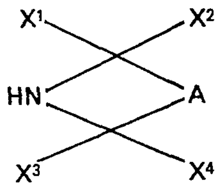

II

in der $X^1$, $X^2$, $X^3$, $X^4$ und A die oben angegebene Bedeutung haben, mit Carbonylverbindungen der Formel III

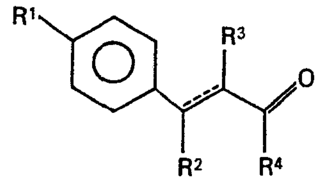

III

in der $R^1$, $R^2$, $R^3$ die angegebene Bedeutung besitzen und die gestrichelte Bindung eine Doppelbindung oder eine einfache Bindung ist in einfacher Weise erhält, wenn man die Umsetzung in Gegenwart von Wasserstoff und einem Hydrierkatalysator durchführt, der Palladium im Gemisch mit Zink, Cadmium, Mangan und/oder einem Oxid der Seltenen Erdmetalle auf einem inerten Träger enthält. Der inerte Träger kann beispielsweise aus Aluminiumoxid, Kieselsäure oder Aktivkohle bestehen. Man führt die Umsetzung beispielsweise bei Temperaturen zwischen 10 und 200°C durch. Es kann drucklos oder bei Drucken bis zu 300 bar gearbeitet werden.

Der Vorteil des erfindungsgemässen Verfahrens besteht darin, dass insbesondere empfindliche Carbonylverbindungen, wie $\alpha$, $\beta$-ungesättigte Aldehyde oder Ketone, die die Amine an die Doppelbindungen anlagern könnten und dann keine einheitlichen Endprodukte ergeben könnten, oder Aldehyde bzw. Ketone, die Aldolkondensationen eingehen könnten, verwendet werden können und einheitliche Endprodukte ergeben. Ein weiterer Vorteil des erfindungsgemässen Verfahrens ist darin zu sehen, dass die gewünschten Endprodukte der Formel I aufgrund der hohen Selektivität der verwendeten Katalysatoren in sehr sauberer Form anfallen. So werden die bei diesem Reaktionstyp zu erwartenden Nebenprodukte, wie beispielsweise die durch Hydrierung aus den Carbonylverbindungen zu erwartenden Alkohole der Formel

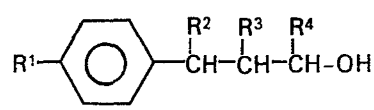

oder ungesättigte Amine der Formeln

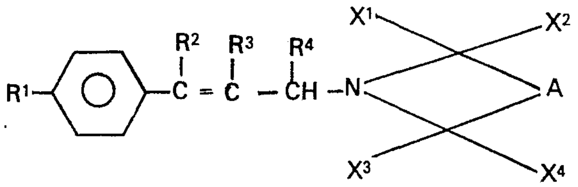

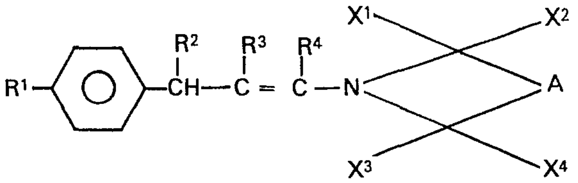

kaum erhalten.

Bei der Verwendung eines weniger selektiven Katalysators, beispielsweise des bekannten Palladium-Silber-Katalysators (DE-PS 1 179 947, DE-OS 2 118 283) werden die angegebenen Nebenprodukte in erheblich grösserer Menge erhalten (vgl. Beispiele 13 + 18).

Beim Arbeiten nach dem erfindungsgemässen Verfahren hat man weiterhin den Vorteil, dass sterisch einheitliche Produkte erhalten werden, wenn sterisch einheitliche Produkte als Ausgangsprodukte verwendet werden. Die an Hydrierkatalysatoren oft autretenden Isomerisierungen (Houben-Weyl, «Methoden der Organischen Chemie», 4/2, S. 276 bis 283) werden hier nicht beobachtet.

Die Aminkomponente kann nach dem erfindungsgemässen Verfahren in stöchiometrischer Menge, bezogen auf die Carbonylverbindung oder in einem bis zu 10fach molaren Überschuss angewendet werden.

Als Ausgangsprodukte der Formel III kommen beispielsweise folgende Carbonylverbindungen in Frage:

3-Phenyl-2-methyl-prop-2-enal, 3-Phenyl-2-methyl-propanal, 3-Phenyl-3-methyl-prop-2-enal, 3-Phenyl-3-methyl-propanal, 4-Phenyl-but-3-en-2-on, 4-Phenyl-butan-2-on, 3-(4′-Methyl-phenyl)-2-methyl-prop-2-enal, 3-(4′-Methylphenyl)-2-methylpropanal, 3-(4′Isopropylphenyl)-2-methyl-prop-2-enal, 3-(4′-Isopropylphenyl)-2-methyl-propanal, 3-(4′-Tertiärbutylphenyl)-2-methyl-prop-2-enal, 3-(4′-Tertiärbutylphenyl)-2-methyl-propanal, 3-Phenyl-2-äthyl-prop-2-enal, 3-Phenyl-2-äthylpropanal, 3-Phenyl-2-isopropyl-prop-2-enal, 3-Phenyl-2-ispopropyl-propanal, 3-)4′-Isopropylphenyl)-3-methyl-prop-2-enal, 3-(4′-Isopropylphenyl)-3-methylpropanal, 3-(4′-Tertiärbutylphenyl)-3-methyl-prop-2-enal, 3-(4′-Tertiärbutylphenyl)-3-methyl-propanal, 4-(4′-Tertiärbutylphenyl)-but-3-en-2-on, 4-(4′-Tertiärbutylphenyl)-buten-2-on, 3-(4′-Methoxyphenyl)-2-methyl-prop-2-enal, 3-(4′-Methoxyphenyl)-2-methyl-propanal, 3-(4′-Isopropoxyphenyl)-2-methyl-prop-2-enal, 3-(4′-Isopropopoxyphenyl)-2-methyl-propanal, 3-(4′-Cyclohexylphenyl)-2-methyl-prop-2-enal, 3-(4′-Cyclohexylphenyl)-2-methyl-propanal, 3-(4′-Cyclopentylphenyl)-2-methyl-prop-2-enal, 3-(4′-Cyclopentylphenyl)-2-methyl-propanal.

Die Herstellung der bekannten Ausgangsprodukte III kann durch Aldolkondensation von entsprechend substituierten Benzaldehyden mit aliphatischen Aldehyden bzw. Ketonen erfolgen. Die hydrierten Verbindungen erhält man aus den ungesättigten Produkten durch Partialhydrierung der Kohlenstoff-Kohlenstoff-Doppelbindung (US-PS 2 976 321).

Als tertiäre Amine der Formel II, die nach dem erfindungsgemässen Verfahren umgesetzt werden können, sind beispielsweise folgende Verbindungen zu nennen:

Pyrrolidin, Piperidin, 2-Methylpiperidin, 3-Methylpiperidin, 4-Methylpiperidin, 2,6-Dimethylpiperidin, 3,5-Dimethylpiperidin, 4-Äthylpiperidin, Morpholin, 2-Methylmorpholin, 2-Äthylmorpholin, 3-Methylmorpholin, 2,6-Dimethylmorpholin, 3,5-Dimethylmorpholin, 2,5-Dimethylmorpholin, 2,6-Diäthylmorpholin, Piperazin, 1-Methylpiperazin, Hexamethylenimin, 2,3-Dimethylhexamethylenimin, 3,5,5-Trimethylhexamethylenimin und 3,3,5-Trimethylhexamethylenimin.

Die Umsetzung kann ohne Lösungsmittel oder in Anwesenheit von Lösungsmitteln, die unter den Reaktionsbedingungen inert sind, durchgeführt werden. Als Lösungsmittel kommen beispielsweise in Frage:

Methanol, Äthanol, Propanol, Tetrahydrofuran, Dioxan, Anisol, Äthylenglykol-monomethyläther, 1,2-Dimethoxyäthan, Methyl-tert.-butyläther, Cyclohexylmethyläther, Di-n-butyläther, Toluol, Cyclohexan.

Die Umsetzung ist sowohl kontinuierlich als auch diskontinuierlich – bevorzugt in flüssiger Phase – durchführbar.

Das nach dem Verfahren der Erfindung verwendete Katalysatorsystem enthält einerseits Palladium als Hydriermetall und andererseits die Zusatzkomponenten Zink, Cadium, Mangan oder ein Oxid der Seltenen Erdmetalle oder Gemische von Oxiden der Seltenen Erdmetalle, wobei die genannten Zusatzkomponenten allein oder im Gemisch miteinander neben dem Palladium vorliegen können. Diese Katalysatorzusätze haben eine Erhöhung der Selektivität des Hydrierkatalysators zur Folge.

Als Oxide der Seltenen Erdmetalle bezeichnen wir erfindungsgemäss die Oxide von Lanthan (La), Cer (Ce), Praseodym (Pr), Neodym (Nd), Samarium (Sm), Europium (Eu), Thulium (Tm), Ytterbium (Yb), Lutetium (Lu). Bevorzugt seien folgende Oxide der Seltenen Erden genannt: $La_2O_3$, $Pr_2O_2$ und $Nd_2O_3$.

In dem erfindungsgemäss verwendeten Katalysatorsystem können die Oxide der Seltenen Erdmetalle in reiner Form oder als Gemisch der Oxide mehrerer Seltener Erdmetalle, so wie es technisch anfällt, vorliegen.

Als inerte Träger sind beispielsweise neben den bereits genannten $Al_2O_3$, $SiO_2$ und Aktivkohle noch Aluminiumsilikate und Magnesiumsilikate zu nennen.

Der Palladiumgehalt des Katalysators, bezogen auf das Trägermaterial, ist nicht kritisch und kann in weiten Grenzen variiert werden. Zweckmässig

ist ein Gehalt von 0,05 bis 15 Gew.-%. Für die Zusatzkomponenten des Katalysators (Zink, Cadmium, Mangan, Oxide der Seltenen Erdmetalle) ist ein Gehalt von 0,01 bis 10 Gew.-% – bezogen auf den Träger – zweckmässig. Das Gewichtsverhältnis der Zusatzkomponenten des Katalysators zu dem Palladiummetall kann beispielsweise zwischen 400:1 bis 1:150, bevorzugt bei 50:1 bis 1:10 liegen. Man verwendet den Katalysator beispielsweise in Strängen von 5 mm Durchmesser und 10 mm Länge oder als Pulver.

Die nach dem Verfahren der Erfindung hergestellten Verbindungen sind bekannt und können als Zwischenprodukte für Pflanzenschutzmittel oder als Wirkstoffe in bekannten Pflanzenschutzmitteln Verwendung finden (DE-OS 2752096, 2752135, 2656747).

Die in den folgenden Beispielen genannten Teile bedeuten Gew.-Teile, Raumteile verhalten sich zu ihnen wie Liter zu Kilogramm.

Beispiel 1

In ein zylindrisches Reaktionsrohr mit einem Volumen von 500 Raumteilen wird ein Katalysator mit der Zusammensetzung von 0,5 Gew.-% Pd, 5 Gew.-% $Pr_2O_3$ (95%iges $Pr_2O_3$, Rest besteht aus Oxiden anderer Seltener Erdmetalle) auf $Al_2O_3$ als Träger eingefüllt und auf 130°C erhitzt. Über dieses Katalysatorbett werden stündlich 60 Teile einer Mischung geführt, die aus 146 Teilen 3-Phenyl-2-methyl-prop-2-enal (α-Methylzimtaldehyd) und 115 Teilen cis-2,6-Dimethylmorpholin besteht. Gleichzeitig werden 100000 Raumteile Wasserstoff bei einem Druck von 50 bar durch das Reaktionsrohr im Gleichstrom hindurchgeleitet. Das aus dem Reaktionsrohr herausfliessende Reaktionsprodukt wird unter Druck abgekühlt und dann entspannt. Es fallen hierbei stündlich 60 Teile Rohprodukt an, die destillativ gereinigt werden. Die Destillation von 100 Teilen Rohprodukt führt zu 77,5 Teilen N-(3′-Phenyl-2′-methylpropyl)-cis-2,6-dimethylmorpholin, $Kp_{0,01}$ = 95°C. Das entspricht einer Ausbeute von 83% d. Th.

Beispiel 2

In die gleiche Apparatur wie in Beispiel 1 beschrieben wird ein Katalysator mit der Zusammensetzung von 0,50 Gew.-% Pd, 0,11 Gew.-% Zn und 0,10 Gew.-% Cd auf $Al_2O_3$ als Träger eingefüllt und auf 100°C erhitzt. Über dieses Katalysatorbett werden stündlich 60 Teile einer Mischung geführt, die aus 146 Teilen 3-Phenyl-2-methyl-prop-2-enal (α-Methylzimtaldehyd) und 115 Teilen cis-2,6-Dimethylmorpholin besteht. Gleichzeitig werden 10000 Raumteile Wasserstoff bei einem Druck von 50 bar durch das Reaktionsrohr im Gleichstrom hindurchgeleitet. Das aus dem Reaktionsrohr herausfliessende Produkt wird unter Druck abgekühlt und dann entspannt. Es fallen hierbei stündlich 60 Teile Rohprodukt an, die destillativ gereinigt werden. Die Destillation von 100 Teilen Rohprodukt führt zu 75,5 Teilen N-(3′Phenyl-2′-methylpropyl)-cis-2,6-dimethylmorpholin, entspr. 81% d. Th.

Beispiel 3

In der gleichen Apparatur und mit dem gleichen Katalysator, wie in Beispiel 1 beschrieben, wird bei einer stündlichen Zufuhr von 120 Teilen einer Mischung, die aus 261 Teilen Methanol, 146 Teilen 3-Phenyl-2-methyl-prop-2-enal (α-Methylzimtaldehyd) und 115 Teilen 2,6-Dimethylmorpholin mit 75 Gew.-% cis-und 25 Gew.-% trans-Verbindung besteht, die Umsetzung bei 120°C vorgenommen. Gleichzeitig mit der vorher genannten Mischung werden 100000 Raumteile Wasserstoff bei einem Druck von 50 bar durch das Reaktionsrohr im Gleichstrom hindurch geleitet. Das aus dem Reaktionsrohr herausfliessende Reaktionsprodukt wird unter Druck abgekühlt und dann entspannt. Es fallen hierbei stündlich 120 Teile Rohprodukt an, die destillativ gereinigt werden. Die Destillation von 200 Teilen Rohprodukt führt zu 80,5 Teilen N-(3′Phenyl-2′-methylpropyl)-2,6-dimethylmorpholin mit 75 Gew.-% cis- und 25 Gew.-% trans-Verbindung, $KP_{12}$ = 155 bis 157°C. Das entspricht einer Ausbeute von 86% d. Th.

Beispiel 4

In der gleichen Apparatur und mit dem gleichen Katalysator wie in Beispiel 1 beschrieben, wird bei einer stündlichen Zufuhr von 120 Teilen einer Mischung, die aus 317 Teilen Methanol, 202 Teilen 3-p-Tertiärbutylphenyl-2-methyl-prop-2-enal und 115 Teilen cis-2,6-Dimethylmorpholin besteht, die Umsetzung bei 130°C vorgenommen. Gleichzeitig mit der vorher genannten Mischung werden 100000 Raumteile Wasserstoff bei einem Druck von 50 bar durch das Reaktionsrohr im Gleichstrom hindurchgeleitet. Das aus dem Reaktionsrohr herausfliessende Reaktionsprodukt wird unter Druck abgekühlt und dann entspannt. Es fallen hierbei stündlich 120 Teile Rohprodukt an, die destillativ gereinigt werden. Die Destillation von 200 Teilen Rohprodukt führt zu 88,5 Teilen N-(3′-(p-Tertiärbutylphenyl)-2′-methyl-propyl)-cis-2,6-dimethylmorpholin, $KP_{18}$ = 206°C. Das entspricht einer Ausbeute von 92,5% d. Th.

Beispiel 5

In einem Rührautoklaven von 1000 Raumteilen Inhalt wird eine Mischung aus 153 Teilen 3-p-Tertiärbutylphenyl-2-methyl-propanal, 70 Teilen Piperidin, 230 Teilen Methanol und 20 Teilen Katalysator, der aus 0,5 Gew.-% Pd, 5 Gew.-% $Nd_2O_3$ auf $Al_2O_3$ besteht, bei 70°C und 50 bar Wasserstoffdruck bis zur Druckkonstanz hydriert. Anschliessend lässt man den Autoklaven abkühlen, filtriert den Katalysator ab und reinigt das Filtrat destillativ. Man erhält 195 Teile N-(3′-(p-Tertiärbutylphenyl)-2′-methyl-propyl)-piperidin, $Kp_{0,2}$ = 117°C. Die Ausbeute beträgt 94% d. Th.

Beispiel 6

In der in Beispiel 5 näher beschriebenen Apparatur wird ein Gemisch aus 153 Teilen 4-(p-Tertiärbutylphenyl)-butan-2-on, 115 Teilen 2,6-Dimethylmorpholin (75% cis-/25% trans-Verbindung),

270 Teilen Methanol und 20 Teilen des unter Beispiel 5 näher beschriebenen Katalysators bei 140°C und 50 bar Wasserstoffdruck bis zur Druckkonstanz hydriert. Anschliessend lässt man den Autoklaven abkühlen, filtriert den Katalysator ab und reinigt das Filtrat destillativ. Man erhält 195,5 Teile N-(4'-(p-Tertiärbutylphenyl)-but-2'-yl)-2,6-dimethylmorpholin (75% cis-/25% trans-Verbindung), $Kp_{0,01}$ = 143°C. Die Ausbeute beträgt 86% d. Th.

Beispiel 7

Man verfährt analog Beispiel 5, verwendet aber als Aminkomponente 2,2,4-Trimethylazetidin. Hierbei fällt als Endprodukt N-(3'-(p-Tertiärbutylphenyl)-2'-methyl-propyl)-2,2,4-trimethyl-azetidin an, $Kp_{0,3}$ = 134°C. Die Ausbeute beträgt 93% d. Th.

Beispiel 8

Man verfährt analog Beispiel 5, verwendet aber als Ausgangsstoff 3-p-Tertiärbutylphenyl-2-methyl-prop-2-enal als Carbonylkomponente und 2,3-Dimethylhexamethylenimin als Aminkomponente. Als Endprodukt wird hierbei N-(3'-(p-Tertiärbutylphenyl)-2'-methyl-propyl)-2,3-dimethylhexamethylenimin, $Kp_{0,01}$ = 144°C erhalten. Die Ausbeute beträgt 95% d. Th.

Beispiel 9

Man verfährt analog Beispiel 5, verwendet aber als Aminkomponente Pyrrolidin. Als Endprodukt fällt N-(3'-(p-Tertiärbutylphenyl)-2'-methyl-propyl)-pyrrolidin an, $Kp_{0,3}$ = 115 °C. Die Ausbeute beträgt 96% d. Th.

Beispiel 10

In einem Rührautoklaven von 1000 Raumteilen Inhalt wird eine Mischung aus 190 Teilen 3-p-Isopropylphenyl-3-methyl-propanal, 111 Teilen 2-Methylmorpholin, 300 Teilen Methanol und 20 Teilen Katalysator, der aus 0,5 Gew.-% Pd, 0,11 Gew.-% Zn und 0,1 Gew.-% Cd auf $Al_2O3$ besteht, bei 120°C und 50 bar Wasserstoffdruck bis zur Druckkonstanz hydriert. Anschliessend lässt man den Autoklaven abkühlen, filtriert den Katalysator ab und reinigt das Filtrat destillativ. Man erhält 266 Teile N-(3'-(p-Isopropylphenyl)-3'-methylpropyl)-2-methylmorpholin, $Kp_{0,05}$ = 122°C. Die Ausbeute beträgt 82% d. Th.

Beispiel 11

Man verfährt analog Beispiel 10, verwendet aber als Ausgangsstoff 3-p-Methoxyphenyl-2-methyl-propanal als Carbonylkomponente und cis-2,6-Dimethylmorpholin als Aminkomponente. Dies führt zu N-(3'-p-Methoxyphenyl)-2'-methylpropyl)-cis-2,6-dimethylmorpholin, $Kp_{0,1}$ = 129°C als Endprodukt. Die Ausbeute beträgt 82% d. Th.

Beispiel 12

In einer Rührapparatur mit einem Inhalt von 2000 Raumteilen wird ein Gemisch aus 101 Teilen 3-p-Tertiärbutylphenyl-2-methyl-prop-2-enal und 61 Teilen cis-2,6-Dimethylmorpholin, das in 900 Teilen Methanol gelöst ist, in Gegenwart von 5 Teilen eines Hydrierkatalysators mit der Zusammensetzung von 10 Gew.-% Pd, 0,11 Gew.-% Zn, 0,1 Gew.-% Cd auf $Al_2O_3$ bei 30°C und Normaldruck bis zum Ende der Waserstoffaufnahme hydriert. Der Katalysator wird abfiltriert und das Filtrat destillativ aufgearbeitet. Hierbei werden 141 Teile N-(3'-(p-Tertiärbutylphenyl)-2'-methyl-propyl)-cis-1,6-dimethylmorpholin, $Kp_{18}$ = 206°C erhalten. Das entspricht einer Ausbeute von 93%.

Beispiel 13 – Vergleichsbeispiel

Mit einem Katalysator der Zusammensetzung von 0,36 Gew.-% Pd, 4,8 Gew.-% Ag und 1,05 Gew.-% Mn auf $SiO_2$ wird völlig analog Beispiel 1 mit denselben Einsatzstoffen und unter denselben Reaktionsbedingungen gearbeitet. Das hierbei erhaltene Reaktionsprodukt hat nach gaschromatographischer Analyse folgende Zusammensetzung:

22 Gew.-% N-(3'-Phenyl-2'-methyl-propyl)-cis-2,6-dimethylmorpholin, 14 Gew.-% N-(3'-Phenyl-2'-methyl-prop-2'-enyl)-cis-2,6-dimethylmorpholin, 36,2 Gew.-% 3-Phenyl-2-methylpropanol und 27,8 Gew.-% cis-2,6 Dimethylmorpholin.

Beispiel 14

In einem Rührautoklaven von 5000 Raumteilen Inhalt wird eine Mischung aus 2500 Raumteilen Methanol, 366 Teilen cis-2,6-Dimethylmorpholin, 606 Teilen 3-p-Tertiärbutyl-phenyl-2-methyl-prop-2-enal und 35 Teilen Katalysator, der aus 0,5 Gew.-% Pd, 5 Gew.-% $Pr_2O_3$, 1 Gew.-% Mn und als Rest $Al_2O_3$ besteht, stufenweise zunächst bei 50° und 50 bar Wasserstoffdruck, dann bei 90°C und 50 bar Wasserstoffdruck, zum Schluss bei 120°C und 50 bar Wasserstoffdruck, bis zur Druckkonstanz hydriert. Anschliessend lässt man den Autoklaven abkühlen, filtriert den Katalysator ab und reinigt das Filtrat destillativ. Man erhält 856 Teile N-(3'-p-Tertiärbutylphenyl-2'-methyl-propyl)-cis-2,6-dimethylmorpholin, $Kp_{24\,mbar}$ = 206°C. Das entspricht einer Ausbeute von 94% d. Th. (bez. auf 3-p-tertiärbutylphenyl-2-methyl-prop-2-enal).

Beispiel 15

Ein Rührautoklav von 1000 Raumteilen Inhalt wird mit einer Mischung aus 153 Teilen 3-p-Tertiärbutylphenyl-2-methyl-propanal, 75 Teilen Hexamethylenimin, 230 Teilen Methanol und 20 Teilen Katalysator, der aus 0,5% Pd, 5% $Pr_2O_3$, 1% Mn auf $Al_2O_3$ besteht, gefüllt. Nun wird bei 90°C und 50 bar Wasserstoffdruck bis zur Druckkonstanz hydriert. Danach lässt man den Autoklaven abkühlen, filtriert den Katalysator ab und reinigt das Filtrat destillativ. Hierbei werden 207 Teile N-[3-(p-Tertiärbutylphenyl)-2'-methyl-propyl]-hexamethylenimin, $Kp_{0,26\,mbar}$ = 130°C, erhalten. Die Ausbeute beträgt 96% d. Th. (bez. auf 3-p-Tertiärbutyl-phenyl-2-methyl-propanal).

Beispiel 16

Man verfährt analog Beispiel 14, verwendet aber als Carbonylkomponente 3-Phenyl-2-methyl-prop-2-enal. Hierbei fällt als Endprodukt N-(3′-Phenyl-2′-methylpropyl)-cis-2,6-dimethylmorpholin an, $Kp_{0,05\ mbar}$ = 95°C. Die Ausbeute beträgt 93% d. Th.

Beispiel 17

Man verfährt analog Beispiel 15, verwendet aber als Aminkomponente 3,5-Dimethylpiperidin. Hierbei fällt als Endprodukt N-[3′-(p-Tertiärbutylphenyl)-2′-methylpropyl]-3,5-dimethylpiperidin an, $Kp_{0,4\ mbar}$ = 135°C.

Beispiel 18 – Vergleichsbeispiel

Mit einem Katalysator der Zusammensetzung von 0,36 Gew.-% Pd, 4,8 Gew.-% Ag und 1,05 Gew.-% Mn auf $SiO_2$ wird völlig analog Beispiel 14 mit denselben Einsatzstoffen und unter denselben Reaktionsbedingungen gearbeitet. Das hierbei erhaltene Reaktionsprodukt hat nach gaschromatographischer Analyse folgende Zusammensetzung:
46 Gew.-% N-(3′-p-Tertiärbutylphenyl-2′-methylpropyl)-cis-2,6-dimethylmorpholin, 25 Gew.-% N-(3′-p-Tertiärbutylphenyl-2′-methyl-prop-2′-enyl)-cis-2,6-dimethylmorpholin, 18 Gew.-% 3-p-Tertiärbutylphenyl-2-methyl-propanol und 11 Gew.-% cis-2,6-Dimethylmorpholin.

**Patentanspruch**

Verfahren zur Herstellung von Aralkylaminen der Formel I

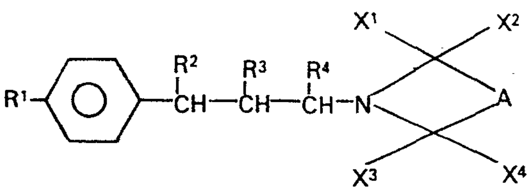
I

in der $R^1$ ein Wasserstoffatom, einen aliphatischen Rest mit 1 bis 10 Kohlenstoffatomen, einen cycloaliphatischen Rest mit 5 bis 7 Kohlenstoffatomen oder eine Alkoxygruppe mit 1 bis 6 Kohlenstoffatomen, $R^2$, $R^3$ und $R^4$ ein Wasserstoffatom oder einen Alkylrest mit 1 bis 4 Kohlensstoffatomen, $X^1$, $X^2$, $X^3$ und $X^4$ ein Wasserstoffatom oder einen Alkylrest mit 1 bis 4 Kohlenstoffatomen, A den Rest

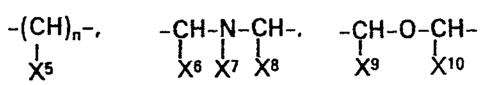

und $X^5$, $X^6$, $X^7$, $X^8$, $X^9$ und $X^{10}$ ein Wasserstoffatom oder einen Alkylrest mit 1 bis 4 Kohlenstoffatomen und n die Zahlen 1, 2, 3 oder 4 bedeutet, durch Umsetzung von sekundären Aminen der Formel II

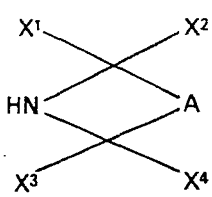
II

in der $X^1$, $X^2$, $X^3$, $X^4$ und A die oben angegebene Bedeutung haben, mit Carbonylverbindungen der Formel III

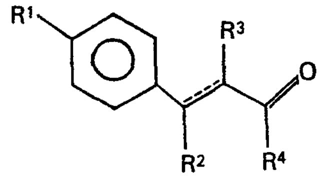
III

in der $R^1$, $R^2$, $R^3$ und $R^4$ die oben angegebene Bedeutung haben und die gestrichelte Bindung eine Doppelbindung oder eine einfache Bindung sein kann, dadurch gekennzeichnet, dass man die Umsetzung in Gegenwart von Wasserstoff und einem Hydrierkatalysator durchführt, der Palladium im Gemisch mit Zink, Cadmium, Mangan und/oder einem Oxid der Seltenen Erdmetalle auf einem inerten Träger enthält.

**Revendication**

Procédé de préparation d'aralkylamides de formule I

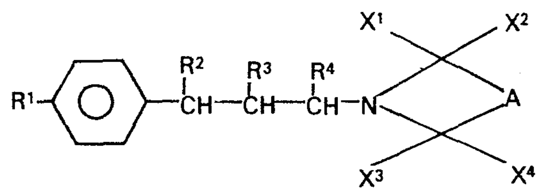
I

dans laquelle $R^1$ représente un atome d'hydrogène, un reste aliphatique à 1 à 10 atomes de carbone, un reste cycloaliphatique à 5 à 7 atomes de carbone ou un groupe alcoxy à 1 à 6 atomes de carbone, $R^2$, $R^3$ et $R^4$ un atome d'hydrogène ou un reste alkyle à 1 à 4 atomes de carbone, $X^1$, $X^2$, $X^3$ et $X^4$ un atome d'hydrogène ou un reste alkyle à 1 à 4 atomes de carbone, A le reste

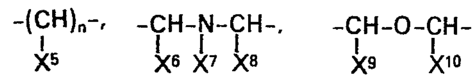

et $X^{10}$ un atome d'hydrogène ou un reste alkyle à 1 à 4 atomes de carbone et n les nombres 1, 2, 3 ou 4, par réaction d'amines secondaires de formule II

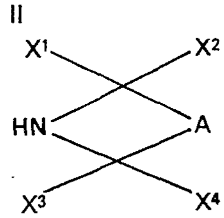
II

dans laquelle $X^1$, $X^2$, $X^3$, $X^4$ et A ont les significations indiquées ci-dessus, avec des composés carbonyles de formule III

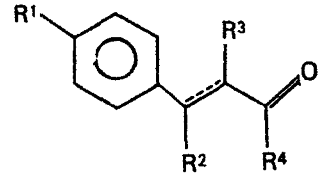
III

dans laquelle $R^1$, $R^2$, $R^3$ et $R^4$ ont la signification indiquée plus haut et la liaison soulignée en pointillé peut être une double liaison ou une liaison simple, caractérisé par le fait que l'on effectue la réaction en présence d'hydrogène et d'un catalyseur d'hydrogénation qui contient, sur un support inerte, du palladium en mélange avec du zinc, du cadmium, du manganèse et/ou un oxyde des terres rares.

**Claim**

A process for the preparation of an aralkylamine of the formula I

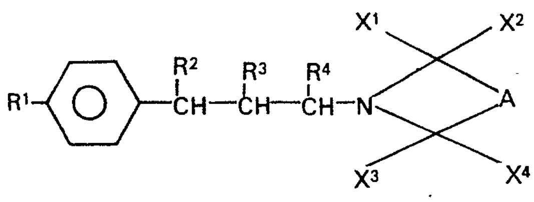

I

where $R^1$ is hydrogen, an aliphatic radical of 1 to 10 carbon atoms, a cycloaliphatic radical of 5 to 7 carbon atoms or alkoxy of 1 to 6 carbon atoms,

$R^2$, $R^3$ and $R^4$ are hydrogen or alkyl of 1 to 4 carbon atoms,

$X^1$, $X^2$, $X^3$ and $X^4$ are hydrogen or alkyl of 1 to 4 carbon atoms,

A is 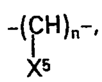 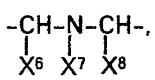 or 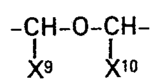

$X^5$, $X^6$, $X^7$, $X^8$, $X^9$ and $X^{10}$ are hydrogen or alkyl of 1 to 4 carbon atoms, and

n is 1, 2, 3, or 4

by reacting a secondary amine of the formula II

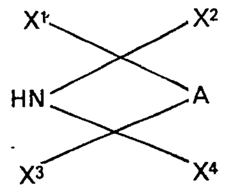

II

where $X^1$, $X^2$, $X^3$, $X^4$ and A have the above meanings, with a carbonyl compound of the formula III

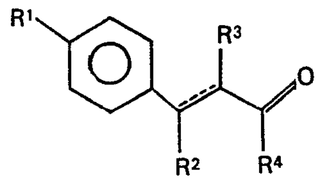

III

where $R^1$, $R^2$, $R^3$ and $R^4$ have the above meanings, and the bond shown in broken lines may be a double bond or a single bond, characterized in that the reaction is carried out in the presence of hydrogen and of a hydrogenation catalyst which comprises palladium, mixed with zinc, cadmium, manganese and/or a rare earth metal oxide, on an inert carrier.